(19) Europäisches Patentamt
European Patent Office
Office européen des brevets

(11) **EP 4 254 425 A1**

(12) **EUROPEAN PATENT APPLICATION**

(43) Date of publication:
**04.10.2023 Bulletin 2023/40**

(21) Application number: **22165124.3**

(22) Date of filing: **29.03.2022**

(51) International Patent Classification (IPC):
**G16H 50/30** (2018.01)    **A61B 5/00** (2006.01)
**G16H 40/63** (2018.01)    **G16H 40/67** (2018.01)
**A61B 5/024** (2006.01)

(52) Cooperative Patent Classification (CPC):
**G16H 50/30; A61B 5/1102; G16H 40/63;
G16H 40/67;** A61B 5/02438; A61B 5/02444;
A61B 5/0816; A61B 5/318; A61B 5/7235;
A61B 5/7267; G16H 10/60; G16H 50/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Heartkinetics**
**6041 Gosselies (BE)**

(72) Inventors:
• **Migeotte, Pierre-François**
**1000 Bruxelles (BE)**
• **Gorlier, Damien**
**7800 Ath (BE)**

(74) Representative: **Calysta NV**
**Lambroekstraat 5a**
**1831 Diegem (BE)**

(54) **METHOD, SYSTEM AND COMPUTER PROGRAM FOR DETECTING A HEART HEALTH STATE**

(57)    Method for detecting $\alpha$ health state of $\alpha$ heart of $\alpha$ user comprising the following steps: receiving, in $\alpha$ processing means, $\alpha$ measurement signal from an IMU placed on $\alpha$ body of the user; determining, in the process- ing means, $\alpha$ plurality of features based on the measurement signal; and determining, in an AI engine of the processing means, based on the plurality of features the health state of the heart.

**Fig. 1**

## Description

### Technical Field

The present invention relates to a method, system and computer program for detecting a health state of a heart of a user, preferably for detecting congestive heart failure (CHF).

### Prior art

The health state of the heart of a human is essential for its well being. The early detection of problems with the heart can avoid serious complications with the human health. Such problems can be CHF, coronary artery disease, atrial fibrillation, high blood pressure, etc. Traditionally heart monitoring is done with an electrocardiogram (ECG). Some heart monitoring techniques are based on a ballistocardiography (BCG) or a seismocardiography (SCG). The SCG is normally measured on the chest of the human. The BCG can be measured in many different ways. While many SCG or BCG measure or use only one or few dimensions, WO201736887 proposes to base the heart monitoring on a combination of ECG and BCG measured at the centre of gravity of the body, eventually combined with an SCG. It is proposed to use multi-dimensional BCG and SCG measuring with the accelerometer three linear dimensions to obtain the linear kinetic energy of the movement of the heart and with the gyroscope three rotational dimensions to obtain the rotation kinetic energy of the movement of the heart. However, these solutions require all complex and special clinical measurement equipment like ECG, SCG and/or BCG equipment which increase the cost of such heart monitoring and makes such heart monitoring not suitable for an easy-at-home heart check which can be performed without medical assistance.

Some state of the art proposes the use of daily life technology to measure SCG of the heart and artificial intelligence (AI) technology to analyze the SCG in order to make simple heart monitoring available to everyone.

US20210057101 discloses to measure a BCG with a remote sensor installed in a flat and analyze the BCG with an AI engine to detect CHF. However, the remote detection of BCG is not very reliable, and the features used for the AI analysis are not disclosed.

EP3267886 proposes to input a 6-dimensional SCG signal into an AI engine to determine cardiac malfunction like atrial fibrillation. To reduce the dimension of the 6-dimensional SCG signal, a principal component analysis is proposed. The principal component analysis might reduce the dimensionality of the signal but does not solve the problem of large number of samples in the time dimension which make the AI analysis computationally complex and the output non-reliable.

EP3897355 proposes heart monitoring to detect internal bleeding in the body. It is disclosed to analyze a BCG and ECG with an AI engine to determine the internal bleeding of the body. The BCG is measured at the feed of the body. Thus, this solution also requires special equipment for heart monitoring.

EP3895605 discloses to monitor a heart with any kind of cardiac data like ECG, electromyography, BCG, or others. The data is subdivided in many sub-periods of around 30sec to 2 min. In each sub-period a feature vector is calculated which is input in an AI engine. The AI engine detects the sub-periods interesting for further human analysis, but not a health state of the heart.

EP3731746 discloses to use a smartphone equipped with a capacitive electrode to measure the SCG and the ECG of the user. The ECG is used to detect the heartbeat periods in the measured signals and to segment each heartbeat period into sub-segments like the systole segment and the diastole segment or even finer sub-segments. Features are extracted from the different sub-segments of the heartbeat period to feed an AI engine to detect cardiac abnormality. As features many potential time or frequency features are suggested. It is mentioned that the cardiac abnormality detection can also work without ECG, but it is not explained how the heartbeat period and the sub-segments are identified without ECG.

However, most of the above solutions require special measurement equipment which makes such a heart monitoring in the daily life not practical. Most solutions need an ECG measurement which is complicated to obtain without an ECG device. Solutions just based on an SCG measurement remain vague about the feature vector used to feed the AI engine. Thus, there is currently no solution known which provides a simple and robust detection of the health state of the heart.

### Brief summary of the invention

It is the object of the invention to provide a method, system and computer program for detecting a health state of a heart of a user which overcomes the problems of the state of the art.

According to the invention, this object is solved by the method, system and computer program according to the independent claims.

According to the invention, this object is solved by a method for detecting a health state of a heart of a user comprising the following steps: receiving, in a processing means, a measurement signal from an IMU placed on a body of the user; determining, in the processing means, a plurality of features based on the measurement signal; determine, in an AI engine of the processing means, based on the plurality of features the health state of the heart.

The use of AI allows to detect the heart health state independently from ECG measurements and complex clinical equipment, e.g. with a simple smartphone.

The dependent claims refer to further advantageous embodiments.

In one embodiment, the IMU is arranged in a smartphone, wherein the plurality of features is determined independently from an ECG measurement, wherein the measurement signal comprises a six-dimensional signal with three linear dimensions representing the linear acceleration of the heart measured with an accelerometer of the IMU and with three rotational dimensions representing the angular velocity of the heart measured with a gyroscope of the IMU, wherein a heartbeat period of the heart of the user is identified based on the measurement signal, wherein a plurality of sub-periods in the heartbeat period are determined, wherein the plurality of features comprise linear features and rotational features, wherein the linear features comprise the values of at least one linear feature variable of the movement of the heart in each sub-period calculated based on the three linear dimensions of the measurement signal, wherein the rotational features comprise the values of at least one rotational feature variable of the movement of the heart in each sub-period calculated based on the three rotational dimensions of the measurement signal. The combination of AI, six-dimensional IMU measurement by a smartphone, ECG independence and the robustness of the rotational and linear parameters versus the orientation of the smartphone allow a good heart state detection with a standard smartphone which just needs to run a software on the smartphone to measure the six-dimensional measurement signal with the IMU of the smartphone. Even if this combination of features is particularly effective, several features can be used also individually or in other sub-combinations.

In one embodiment, the measurement signal comprises a six-dimensional signal with three linear dimensions representing the linear acceleration of the heart measured with an accelerometer of the IMU and with three rotational dimensions representing the angular velocity of the heart measured with a gyroscope of the IMU, wherein a heartbeat period of the heart of the user is identified based on the measurement signal, wherein a plurality of sub-periods in the heartbeat period are determined, wherein the plurality of features comprise linear features and rotational features, wherein the linear features comprise the values of at least one linear feature variable of the movement of the heart in each sub-period calculated based on the three linear dimensions of the measurement signal, wherein the rotational features comprise the values of at least one rotational feature variable of the movement of the heart in each sub-period calculated based on the three rotational dimensions of the measurement signal. Having a combined/scalar linear value, and one combined/scalar rotational value allows to remove the dependence of the measurement signal from the orientation of the IMU. The dependence of the measured components on the orientation of the IMU showed to increase the error rate of the AI engine.

In one embodiment, the plurality of features comprises values of a linear feature variable at different times of one heartbeat and values of a second feature variable at different times of one heartbeat, wherein the first feature variable is determined based on the three linear dimensions, wherein the second feature variable is determined based on the three rotational dimensions. Having a combined/scalar linear value, and one combined/scalar rotational value allows to remove the dependence of the measurement signal from the orientation of the IMU. The dependence of the measured components on the orientation of the IMU showed to increase the error rate of the AI engine.

In one embodiment, the (at least one) linear feature variable is one or more of: the linear (cardiac) power, the linear (cardiac) kinetic energy, and the linear (cardiac) work. In one embodiment, the (at least one) rotational feature variable is one or more of: the rotational (cardiac) power, the rotational (cardiac) kinetic energy, and the rotational (cardiac) work. These kinetic parameters are independent from the orientation of the sensor and proved to be very robust in detecting a heart health state.

In one embodiment, the measurement signal comprises a six-dimensional signal with three linear dimensions representing the linear acceleration of the heart measured with an accelerometer of the IMU and with three rotational dimensions representing the angular velocity of the heart measured with a gyroscope of the IMU, wherein a heartbeat period of the heart of the user is identified, wherein a plurality of sub-periods in the heartbeat period are determined, wherein the plurality of features comprise linear features and rotational features, wherein the linear features comprise the values of the linear kinetic energy of the movement of the heart in each sub-period calculated based on the three linear dimensions of the measurement signal, wherein the rotational features comprise the values of the rotational kinetic energy of the movement of the heart in each sub-period calculated based on the three rotational dimensions of the measurement signal.

In one embodiment, the measurement signal comprises a six-dimensional signal with three linear dimensions representing the linear acceleration of the heart measured with an accelerometer of the IMU and with three rotational dimensions representing the angular velocity of the heart measured with a gyroscope of the IMU. The inventor found out that measurement signals with all six dimensions bring significantly better results, because information can not be hidden in some non-measured dimension. The state of the art often measured only some dimensions or at least considered only some dimensions for the analysis.

In one embodiment, a linear velocity signal is calculated based on the three linear dimensions and the plurality of features comprise at least one linear feature calculated based on the linear velocity signal; and/or a rotational acceleration signal is calculated based on the three rotational dimensions and the plurality of features comprise at least one rotational feature calculated based on the rotational acceleration signal. It was found out that kinetic features based on the linear velocity and/or based on the rotational acceleration which are not directly measured contain many valuable information not available from the measurement signal. Features for the AI engine using these features proved to be more robust over feature using just signal characteristics of the signal measured in the IMU. In particular, the linear kinetic energy computed from the linear velocity signal was found to be of particular interest as feature for the AI engine.

In one embodiment, the method comprises the step of determining, in the processing means, based on the measurement signal a velocity signal representing the velocity of the kinetic movement of the heart, wherein the processing means determines the plurality of features based on the velocity signal.

In one embodiment, the processing means determines from the velocity signal a processed velocity signal representing the squared velocity of the kinetic movement of the heart or the kinetic energy of the movement of the heart, wherein the processing means determines the plurality of features based on the processed velocity signal.

In one embodiment, the plurality of features comprises the values of at least one feature variable at different times of one heartbeat.

In one embodiment, a heartbeat period of the heart of the user is identified, wherein a plurality of sub-periods in the heartbeat period are determined, wherein the plurality of features comprise values of at least one feature variable in each determined sub-period. The definition of the sub-periods (only) based on the measurement signal from the IMU (and not based on the ECG) allows to create such a segmentation also without and ECG signal. This allows to perform the present feature extraction also in simple smartphones.

In one embodiment, a reference point in the heartbeat period is determined and the sub-periods are defined based on the reference point and based on fixed time widths of the sub-periods. The use of fixed time widths of the sub-periods allows an easy, robust, and repeatable computation of the sub-periods. The sub-periods defined based on physiological parameters are computationally complex and error prone, if only one of the physiological parameters is not correctly detected.

In one embodiment, heartbeat periods in the measurement signal are identified based on a matrix profile motif algorithm applied on a signal based on the measurement signal, wherein the matrix profile motif algorithm identifies one or more reference heartbeat periods as motif in the signal based on the matrix profile. The detection of the heartbeats in an IMU signal is very difficult without the measurement of the ECG signal. The use of the matrix profile allows a robust detection of the heartbeat period at low computational costs.

In one embodiment, a respiration information of the user is detected based on the measurement signal.

In one embodiment, the plurality of features comprises a respiration information of the user. Since the heartbeat depends significantly on the respiration phase/state of the user, this feature as input for the AI engine was found to significantly improve the outcome of the AI engine.

In one embodiment, the plurality of features is determined independently from an ECG measurement. This allows to realize the method in any common smartphone with an IMU.

In one embodiment, the AI engine determines, if the heart shows congestive heart failure.

In one embodiment, the method comprising the steps of placing the IMU on a chest of the user, and measuring the measurement signal with the IMU, when placed on the chest of the user.

In one embodiment, the IMU is included in a smartphone, wherein the processing means requests from an operating system running on the smartphone of what type the smartphone is, wherein the plurality of features depend on the type of smartphone received back, in particular on the weight of the type of smartphone received back. This allows to get any kinetic parameter calibrated by the correct mass or moment of inertia of the smartphone. The calibration with the type of sensor used makes the measurement signal much more comparable

In one embodiment, the user lays down, while a smartphone including the IMU is placed on the chest of the user. While the state of the art proposed mainly sleeves or fixations for fixing the measuring device on the chest or somewhere else on the body, it was found by the inventors that measuring the IMU signal while laying down has several advantages for the measurement quality. The user is not moving and thus movements caused by the movement of the user do not disturb the measurement. The user relaxes while laying down and the measurement signal is always measured in a relaxed state which yields more repeatable signals. And finally, the sleeves holding the devices with the IMU damp the movement and reduce thus the signal quality. Other embodiments according to the present invention are mentioned in the appended claims and the subsequent description of an embodiment of the invention.

## Brief description of the Drawings

Fig. 1 shows an embodiment of a system for detecting a health state of a heart of a user according to the invention.
Fig. 2 shows an embodiment of a method for detecting a health state of a heart of a user according to the invention.
Fig. 3 shows an exemplary embodiment of the feature extraction.
Fig. 4 shows a first embodiment of the system.
Fig. 5 shows a second embodiment of the system.
In the drawings, the same reference numbers have been allocated to the same or analogue element.
Fig. 6 shows an example of the measurement signal, of the respiration signal and pre-feature signals.
Fig. 7 shows an example illustration of some steps of the feature extraction.

## Detailed description of an embodiment of the invention

Other characteristics and advantages of the present invention will be derived from the non-limitative following description, and by making reference to the drawings and the examples.

The system, method and computer program according to the invention detect a health state of a heart (heart health state) of a user 10. The terms body and heart shall refer to the body and heart of the user 10 whose heart health state is detected. The user is normally a human being. However, the user can also be an animal, preferably a mammal animal. The user 10 is called a user, not a patient as the system, method and computer program can be performed by any person and no medical surveillance is necessary. Obviously, this shall not exclude that also medical stuff can perform the system, method and computer program according to the invention on a user 10 (patient in this case). The user 10 shall always be the person whose heart health state shall be detected. The person controlling the system, method and computer program according to the invention is preferably the same user, can however be another person.

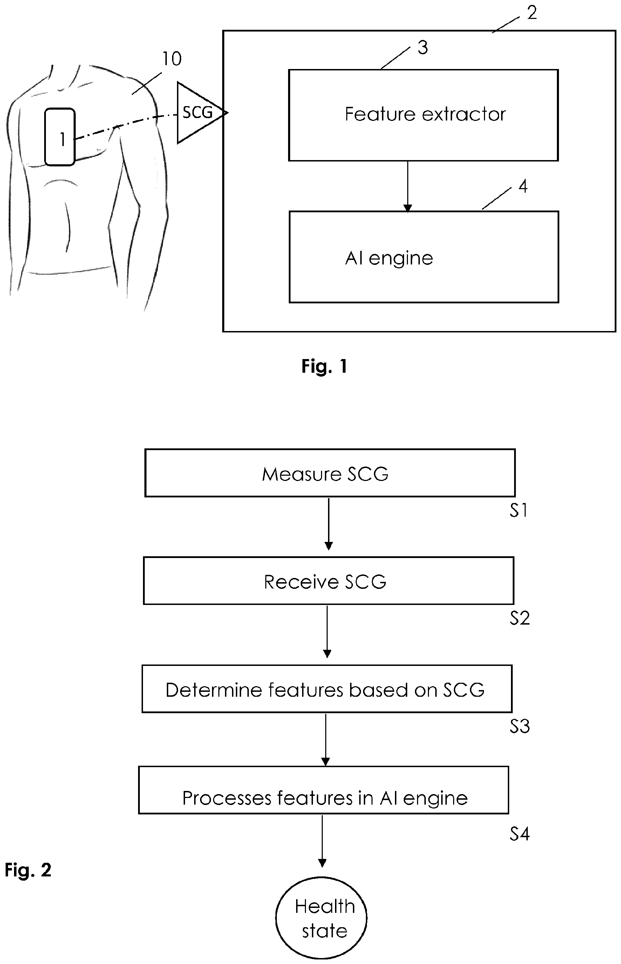

Fig. 1 shows an embodiment of a system for detecting a health state of a heart of a user according to the invention. The system comprises an inertial measurement unit (IMU) 1 and a processing means 2.

The IMU 1 is configured to measure measurement signal for the motion/movement of the heart. The measurement signal comprises preferably at least 6 dimensions. The 6 dimensions comprise 3 translational dimensions also called linear dimensions and 3 rotational dimensions, also called angular dimensions. The IMU 1 comprises normally an accelerometer for measuring the acceleration of the heart movement (for the 3 linear dimensions) and a gyroscope for measuring the angular velocity (for the 3 rotational dimensions). The IMU 1 comprises the accelerometer and the gyroscope normally in a common chip or device. However, the gyroscope and the accelerometer can also be realized as separated chips or devices and would still be considered as IMU 1. The IMU 1 is preferably placed on the chest of the user 10 to measure the measurement signal which then is normally called SCG. Often SCG refers just to the 3 linear acceleration measurements or even to just one linear and/or rotational dimension. But in here SCG shall refer to a six-dimensional measurement signal including the 3 linear dimensions and the 3 rotational dimensions. Normally, one, two or three of the 3 linear dimensions are called SCG, and one, two or three of the 3 rotational dimensions are called Gyrocardiography (GCG). For simplicity, the six-dimensional measurement signal with the SCG and GCG is called in here SCG.

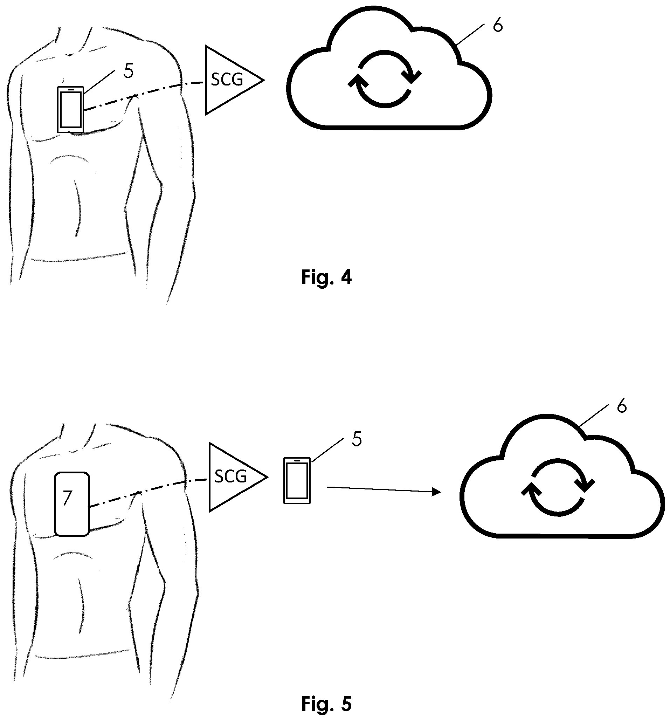

In a preferred embodiment, the IMU 1 is arranged in a smartphone 5 as shown in embodiment of Fig. 4. Thus, the standard IMU 1 available in nearly every smartphone 4 can be used to detect the measurement signal of the heart necessary for the present invention. No other sensor or extra sensor is necessary to perform the measurement of the heart.

However, it is also possible that the IMU 1 is not arranged in a smartphone 5. The IMU 1 can for example be arranged in a wearable 7 worn on the body of the user like a smartwatch, a heart monitor fixed on the chest with a sleeve around the torso, or other wearables. Such a wearable could be connected to a smartphone 5, preferably via wireless connection, e.g. a Bluetooth connection. For example, the IMU 1 can be arranged in a heart monitor which is fixed with a sleeve on the chest of the user 10.

It is also possible that the IMU 1 is arranged in a dedicated device which is not related to a smartphone 5.

The smartphone 5 comprises preferably a smartphone processor, a storage, a communication means, an input means, preferably a touch screen, and an output means, preferably a (touch) screen. The storage of the smartphone 5 is configured to store application programs to perform special functions on the smartphone 5, when the application program is executed on the smartphone processor. Such application programs are often also called apps. The smartphone 5 in the embodiment of Fig. 4 and 5 comprises preferably an application program for performing or requesting at least some steps of the method of the invention described subsequently (subsequently called monitoring app). The monitoring app is preferably installed on the smartphone 5, i.e. stored in the storage of the smartphone 5. However, the term monitoring app shall also refer to a front-end program downloaded each time when accessing with the browser of the smartphone on a certain website. Preferably, the monitoring app is configured to request from the IMU 1 the measurement of the measurement signal and to receive the measurement signal from the IMU 1 (arranged in the same smartphone 5 as in Fig. 4 or connected to the smartphone 5 as in Fig. 5). Preferably, the monitoring app is configured to communicate with a server 6. The communication means is configured to establish a communication connection to a server 6. The communication means can be a WLAN and/or a cell phone network communication module. The cell phone network communication module comprises preferably a Subscriber Identity Module (a virtual one (eSIM), a SIM card or just a holder for such a SIM card) for receiving the data required for communicating with the cellular phone network.

The processing means 2 is configured to perform all or at least some/most steps (S2 to S4) of the invention as described in more detail below.

The processing means 2 can be a single processor or can be a combination of processors. For example, different processors could realize different steps. Preferably, the processing means 2 can comprise a local processor and a remote processor. The local processor can be arranged in the smartphone 5 (smartphone processor) and/or in the wearable 7 or the dedicated device mentioned above. The remote processor can be arranged for example in a server 6 connected to the local processor (or the device including the local processor) via the internet. The remote processor shall not only be understood as one physical processor, but as anything which realizes the functions of the remote processor or server 6 such as for example a server center or a cloud. The steps S2 to S4 described below can be realized all on the local processor or all on the remote processor or distributed over both. Preferably, the processing means comprises a general-purpose processor (or more of them) on which a software is running to realize the functions of the inventive method described later. This software can realize all functions or only some functions. If there is a local processor, there is preferably a local software running on the local processor, e.g. the monitoring app. If there is a remote processor, there is preferably a remote software running on the remote software, e.g. a server or backend software.

The processing means 2 comprises a feature extractor 3 and an AI engine 4.

The feature extractor 3 is configured to realize the step S3 described later to extract the features for the AI engine 4 from the measurement signal. The feature extractor 3 can be realized in the local processor/smartphone 5 or in the remote processor/server 6 or in both. Preferably, the processing means comprises a general-purpose processor (or more of them) on which a software is running to realize the function of the feature extractor 3, e.g. the monitoring app and/or the server software.

The AI engine 4 is configured to realize the step S4 described later. The AI engine 4 can be realized on a general-purpose processor or on a dedicated AI processor. The general-purpose processor can be the same as the one on which the feature extractor 3 is running. In this case, the AI engine 4 is realized as a software. However, the AI engine 4 can also be realized on a special purpose chip or processor specialized for AI applications. Such an AI special purpose chip would then be programmed for the present AI application as described later. The AI engine 4 is preferably arranged on the remote processor/ server 6. However, it would also be possible to have the AI engine 4 on the local processor/smartphone 5.

Fig. 2 shows an embodiment of a method for detecting a health state of a heart of a user according to the invention.

In a first step S1, a measurement signal is measured with the IMU 1 placed on the body of the user 10. The measurement signal is preferably an SCG. The IMU 1 is placed such on the body of the user that the IMU 1 can detect the movement of the heart. Preferably, the IMU 1 is placed on the torso, preferably on the upper portion of the torso and/or preferably on the chest side of the torso, most preferably on the chest of the user 10. Preferably, the user 10 lays down for the measurement and puts the IMU 1, preferably in the form of the smartphone 5, on the torso. Since the user 10 is laying down, the IMU 1 or smartphone 5 must not be fixed on the torso and can freely measure the movement created by the heart. A sleeve or any other means for pushing the IMU 1 against the torso might damp the movement of the IMU 1. In addition, this way the user 10 is in a relaxed state and the measurement is not falsified with other body movements which might falsify the measurement. The user 10 can perform the measurement himself by providing an input command to the monitoring app/smartphone 5/IMU 1 to start the measurement and by placing the smartphone 5/IMU 1 on his torso/chest (in any order). Thus, the measurement can be performed with any common smartphone 5 having an IMU 1 included. The user needs just to download a special monitoring app on the smartphone 5. Preferably, the measurement lasts less than a maximum length, preferably less than 10 minutes, preferably than 5 minutes, preferably than 2 minutes, preferably than 1 minute. Thus, the measurement step can be realized quickly by the user 10. The measurement lasts preferably longer than a minimum length which is preferably longer than one heartbeat, preferably longer than one second, preferably longer than 5 seconds, preferably longer than 10 seconds, preferably longer than 20 seconds. Thus, the IMU 1 records a measurement signal SCG of a certain measurement window. The measurement window is preferably between the minimum length and the maximum length. However obviously other lengths are also possible. Since the IMU 1 /smartphone 5 is placed on the torso of the user 10, it moves with the movement of the heart or the heartbeat. Thus, the measurement signal SCG represents the movement of the heart in the measurement window for six dimensions (degrees of freedom). The measurement signal SCG is measured with a certain sampling rate. The sampling rate is preferably higher than 10Hz, preferably higher than 25 Hz, preferably higher than 50 Hz, preferably higher than 60 Hz, preferably higher than 70 Hz, preferably higher than 80 Hz, preferably higher than 90 Hz, preferably higher than 100 Hz.

Step S1 is actually optional, and the inventive method or computer program could start with step 2 of receiving a measurement signal SCG as described in step S1. If step S1 is included in the method or computer program, the monitoring app/smartphone 5/dedicated device receives preferably a user input to start the measurement with the IMU 1. Once this user input is received, the monitoring app/smartphone 5/dedicated device sends instructions to the IMU 1 to start the measurement signal SCG. The duration of the measurement is preferably predetermined or can be configured in the monitoring app. The monitoring app/smartphone 5/dedicated device preferably gives out an indication that the measurement has ended, when the duration of the measurement has ended, e.g. an audio signal.

In step S2, the system/method/computer program/monitoring app/smartphone 5/processing means 2 receives the measurement signal SCG from the IMU 1.

In step S3, the processing means 2 determines a plurality of features based on the measurement signal SCG. These features are used in the next step as input features (also called feature vector) for the AI engine 4. The features comprise preferably data features derived from the measurement signal SCG and subject features related to the user 10.

The subject features are normally independent from the measurement signal SCG. The subject features comprise preferably one or more of age, sex, weight, height, body mass index (BMI), body surface area (BSA) of the user 10. The subject features are preferably derived from an input information, preferably a user input. The user input can for example be received at the monitoring app in the smartphone 5. However, the input information can also be stored in a database on the server 6 for the user 10 and retrieved from there. For example, this information can be received from a parallel application which already has this information and allows a transfer. The BMI and the BSA can be computed from the weight and height of the user 10. Even if the presence of subject features in the features of the AI engine 4 is preferred, it is also possible to do the step S4 without subject features or with different subject features.

In the preferred embodiment, the data features are derived (only) from the measurement signal SCG measured with the IMU 1, i.e. independent from other sensors arranged on the body, in particular independent from sensors not included in a standard smartphone, in particular independent from an ECG measurement which normally requires special equipment. However, the invention could also be applied in a more complex embodiment including ECG sensors and other sensors.

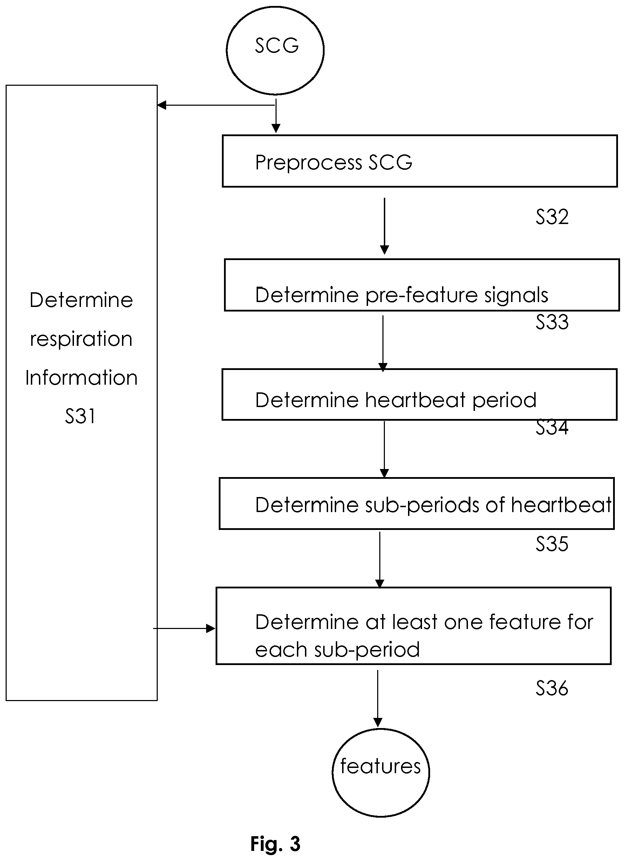

Fig. 3 describes one preferred embodiment to determine the data features based on the measurement signal SCG (subsequently short only SCG). The data features can be obtained of the complete measurement window of the SCG signal or only of a sub-window of the measurement window.

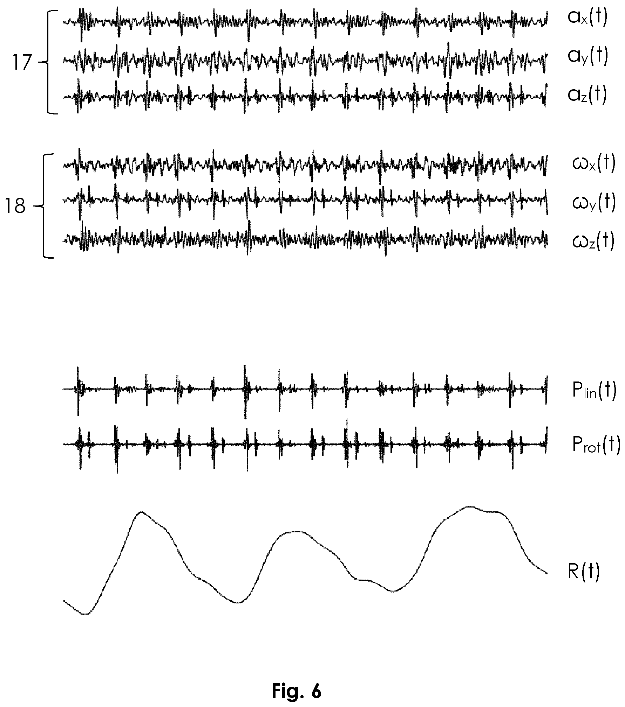

In step S31, respiration information is retrieved from the SCG. Since the respiration causes increase and decrease of the volume of the torso or thorax/chest, the SCG reflects the respiration information as well. Preferably, a respiration signal is extracted from the SCG. This can be realized by independent component analysis, a low pass filter or other measures. The respiration signal is preferably determined based on the complete measurement window. The respiration information is preferably based on the respiration signal. Preferably, the respiration signal is discretized in a number of respiration states. The respiration states include preferably at least four respiration states. The respiration states include preferably two or more of: a high-volume state (HV), a middle volume state during expiration (MV-EX), a low volume state (LV) and a middle volume state during inspiration (MV-IN). There can also be more than four respiration states. The high-volume state HV can be distinguished in two state (high volume during expiration HV-EX and high volume during inspiration LV-IN) or three states (HV-EX, HV, HV-IN). The same can be done for the low volume state LV. Also the middle volume states MV can be made distinguished into smaller states. One respiration cycle/period includes thus a sequence of HV, with subsequent MV-EX, with subsequent LV and with subsequent MV-IN (or other complete sequence of respiration states of a respiration cycle/period). The discretization of the respiration signal leads to a respiration state signal showing the respiration states over time (in the measurement window). The discretization of the respiration signal can be done only on the complete measurement window or only on a sub-window of interest. The features comprise preferably at least one respiration information, preferably the respiration state at at least one time point of a heartbeat. The respiration signal/information can be determined from all six dimensions of the SCG, from a sub-group of dimensions of the SCG or from one or more composite signals determined each from one or more (preferably different) sub-group(s) of the SCG signal. Fig. 6 shows an example of a respiration signal 23 extracted from an example SCG.

In step S32, a preprocessing of the SCG signal is performed. This involves mainly the removal of noise, i.e. removal of signal components not related to the heart movement. This can be done for example by a frequency filter, e.g. a high pass filter or a low pass filter or a band pass filter. The preprocessing step removes preferably frequencies above 100 Hz, preferably above 80Hz, preferably above 70 Hz, preferably above 60 Hz. The preprocessing step removes preferably frequency below 1 Hz, preferably below 2 Hz, preferably below 3 Hz. Preferably, the pre-processing removes also any trend in the SCG. Preprocessing is a preferred step, but is not essential to the invention. The respiration signal/information is preferably determined based on the SCG before preprocessing or with a different preprocessing excluding the removal of the frequencies below normal heartbeat frequencies. Fig. 6 shows an example of the preprocessed six-dimensional SCG signal with the three linear dimensions $\alpha_x(t)$, $\alpha_y(t)$, $\alpha_z(t)$ and the three rotational dimensions $\omega_x(t)$, $\omega_y(t)$, $\omega_z(t)$. The signal $\alpha_x(t)$ shows the acceleration in the x-direction, the signal $\alpha_y(t)$ the acceleration in the y-

direction, the signal $\alpha_z(t)$ the acceleration in the z-direction. The signal $\omega_x(t)$ shows the rotational velocity around the x-direction, the signal $\omega_y(t)$ shows the rotational velocity around the y-direction, the signal $\omega_z(t)$ shows the rotational velocity around the z-direction. The x-direction, y-direction and z-direction refer to the three perpendicular dimensions of a cartesian coordinate system of the IMU 1. Preferably, the coordinate system of the IMU 1 is aligned with the coordinate system of the user 10. The coordinate system of the user has preferably the y-direction in the direction from the feet to the head of the user, the x-direction in the direction from one arm to the other arm, preferably from the right arm to the left arm, and the z-direction from the back of the torso to the chest. Such an alignment could be achieved by giving the user instructions how to place the smartphone 5 on the body. For example, the instructions could show an arrow indicating to the head of the user 10. However, it is also possible that the arrangement of the IMU 1 with respect to the user 10 is not known.

In step S33, at least one pre-feature signal is calculated based on the (preferably pre-processed) SCG signal. In a preferred embodiment, at least one (scalar) linear pre-feature signal is determined based on the combination of the three linear dimensions $\alpha_x(t)$, $\alpha_y(t)$, $\alpha_z(t)$ of the SCG (independent of the three rotational dimensions $\omega_x(t)$, $\omega_y(t)$, $\omega_z(t)$ of the SCG) and/or at least one (scalar) rotational pre-feature signal is determined based on the combination of the three rotational dimensions $\omega_x(t)$, $\omega_y(t)$, $\omega_z(t)$ of the SCG (independent of the three linear dimensions $\alpha_x(t)$, $\alpha_y(t)$, $\alpha_z(t)$ of the SCG). The (linear and/or rotational) pre-feature corresponds preferably to a physical variable describing the movement of the heart like kinetic Energy, kinetic power, work, force/torque, etc. Physical values describing the linear movement of the heart are for example

$$\vec{F} = m\vec{a} \text{ (force vector)} \tag{1}$$

$$K_{lin} = \frac{1}{2}mv^2 \text{ (linear kinetic energy)} \tag{2}$$

$$dw = \vec{F}.\overrightarrow{ds} \quad W_{lin} = \oint \vec{F}.\overrightarrow{ds} \text{ (cardiac linear work)} \tag{3}$$

$$dP_{lin} = \frac{dw}{dt} = \vec{F}.\vec{v} \text{ (cardiac linear power)} \tag{4}$$

with the vector a describing the three-dimensional vector of the linear accelerations at each sample time, with m being the weight of the device containing the IMU 1 (mass), with the vector v being the three-dimensional vector of the linear velocity of the heart at the sample time. A vector is indicated by an arrow above the variable. The force vector F(t) at each sample time t is obtained by the multiplication of the mass m with the acceleration vector $\alpha(t)$ at the respective sample time t $F(t)=(F_x(t), F_y(t), F_z(t))= (m\alpha_x(t), m\alpha_y(t), m\alpha_z(t))$ as shown in equation (1). The acceleration vector $\alpha(t)$ corresponds to the acceleration vector $\alpha(t)=(\alpha_x(t), \alpha_y(t), \alpha_z(t))$ measured with the IMU 1 (and preferably preprocessed in S32). The linear kinetic energy $K_{lin}(t)$ at the sample time t is calculated based on the mass multiplied with the squared linear velocity $v^2(t)$ at the sample time t as shown in equ. (2). The squared linear velocity $v^2(t)$ is preferably calculated based on the squared distance/length of the velocity vector $v=(v_x(t), v_y(t), v_z(t))$ at the sample time t. The distance/length of the velocity vector is preferably the square-root of the sum of the three squared linear components of the velocity $sqrt(v_x^2(t)+ v_y^2(t)+ v_z^2(t))$, i.e. the Euclidian distance metric of the velocity vector v. However, other distance metrics might be used. The velocity vector $v=(v_x(t), v_y(t), v_z(t))$ can be obtained by integrating the acceleration vector $\alpha(t)= (\alpha_x(t), \alpha_y(t), \alpha_z(t))$ from t0, e.g. the start of the measurement signal, until the sample time t. Preferably, the linear velocity signal is calculated (by integration) based on the linear acceleration vector signal. The linear velocity signal can be the linear velocity vector signal with the three linear velocity components $(v_x(t), v_y(t), v_z(t))$ or the length of this linear velocity vector or a function of those. This linear velocity signal can be used to calculate linear pre-feature signals and/or (data) features like the linear kinetic energy. The linear kinetic energy $K_{lin}(t)$ must be distinguished from the signal energy which is often used in the state of the art. For example, the signal energy of the SCG in the x-direction is defined as the sum of the acceleration $\alpha_x^2(t)$ over the measurement signal and has nothing to do with the linear kinetic energy $K_{lin}(t)$ of the movement of the heart at the sample time t. The cardiac linear work $W_{lin}(t1, t2)$ in the time period between t1 and t2 is the integral over the time t between t1 and t2 of the multiplication of the force vector F(t) at the time t and the vector of the change of the path ds(t) at the time t (see equation (3)). The linear kinetic power $dP_{lin}(t)$ at the sample time t (or short only $P_{lin}(t)$) can be calculated by the scalar product of the velocity vector v(t) at the sample time t and the force vector F(t) at the sample time t $P_{lin}(t)= v_x(t) F_x(t) + v_y(t) F_y(t) + v_z(t) F_z(t)$. As well known, these physical variables can be obtained in different ways without deviating from the invention.

Physical values describing the rotational movement of the heart are for example

$$\vec{\tau} = I\vec{\alpha} = \vec{r} \times \vec{F} \text{ (torque)} \tag{5}$$

$$K_{rot} = \frac{1}{2}I\omega^2 \text{ (rotational kinetic energy)} \tag{6}$$

$$dw = \vec{\tau}.\overrightarrow{d\theta} \quad W_{rot} = \oint \vec{\tau}.\overrightarrow{d\theta} \text{ (rotational work)} \tag{7}$$

$$dP_{rot} = \frac{dw}{dt} = \vec{F}.\vec{\omega} \text{ (cardiac rotational power)} \tag{8}$$

with the vector $\alpha(t)$ describing the three-dimensional vector of the rotational/angle accelerations at each sample time, with I being the moment of inertia of the device containing the IMU 1 (mass), with the vector $\omega(t)$ being the three-dimensional vector of the rotational/angular velocity of the heart at the sample time t. The torque vector $\tau(t)$ at each sample time t is obtained by the multiplication of the moment of inertia I with the rotational acceleration vector $\alpha(t)$ at the respective sample time t as shown in equation (5). The angular acceleration vector $\alpha(t)$ corresponds to the first time derivative of the measured (and preferably preprocessed) angular velocity vector $\omega(t)$ with vector $\alpha(t)=(d\omega_x(t)/dt, d\omega_y(t)/dt, d\omega_z(t)/dt)$. The angular/rotational acceleration signal is the signal of the acceleration vector, one of its components or a combination of its components, e.g. its length, over time. The rotational acceleration signal can be used to calculate rotational pre-feature signals. The rotational kinetic energy $K_{rot}(t)$ at the sample time t is calculated based on the moment of inertia (of the device containing the IMU 1) multiplied with the squared rotational velocity $\omega^2(t)$ at the sample time t as shown in equation (6). The squared rotational velocity $v^2(t)$ is preferably calculated based on the squared distance/length of the rotational velocity vector $\omega=(\omega_x(t), \omega_y(t), \omega_z(t))$ at the sample time t. The cardiac rotational work $W_{rot}(t1, t2)$ in the time period between t1 and t2 is the integral over the time t between t1 and t2 of the scalar product of the torque vector $\tau(t)$ at the time t and the vector of the change of the angles $d\theta(t)$ at the time t (see equation (3)). The rotational kinetic power $dP_{rot}(t)$ at the sample time t (or short only $P_{rot}(t)$) can be calculated by the scalar product of the angular velocity vector $\omega(t)$ at the sample time t and the linear force vector F(t) at the sample time t $P_{rot}(t)= \omega_x(t) F_x(t) + \omega_y(t) F_y(t) + \omega_z(t) F_z(t)$. As well known, these physical variables can be obtained in different ways without deviating from the invention.

The measurement of the IMU 1 or the preprocessing step S32 provides the following two measurement sub-signals: the three-dimensional linear acceleration vector signal $\alpha(t)$ and the three-dimensional angular velocity vector signal $\omega(t)$. Preferably in step S33, at least one composite linear kinetic pre-feature signal is computed based on the three-dimensional linear acceleration vector signal $\alpha(t)$ and/or at least one composite rotational kinetic pre-feature signal is computed based on the three-dimensional rotational velocity vector signal $\omega(t)$. The at least one composite linear kinetic pre-feature signal is preferably a scalar value combining the three linear dimensions of the measurement signal in one scalar value, and/or the at least one composite rotational kinetic pre-feature signal is preferably a scalar value combining the three rotational dimensions of the measurement signal in one scalar value so that the composite values are independent of the orientation of the IMU 1, when measuring the measurement signal. The at least one composite linear pre-feature signal comprises preferably the linear kinetic energy signal $K_{lin}(t)$ and/or the linear kinetic power signal $P_{lin}(t)$. One composite linear pre-feature signal and/or the linear kinetic energy signal $K_{lin}(t)$ is preferably calculated/based on the linear velocity vector v(t). The at least one composite rotational pre-feature signal comprises preferably the rotational kinetic energy signal $K_{rot}(t)$ and/or the rotational kinetic power signal $P_{rot}(t)$. One composite linear pre-feature signal and/or the rotation kinetic power signal $P_{rot}(t)$ is preferably calculated/based on the rotational acceleration vector $\alpha(t)$.

The movement of the heart is approximated here by the movement of the IMU 1. So, the physical variables of the movement of the heart correspond at the end to the (preferably preprocessed) movement of the IMU 1 or the device containing the IMU 1. The values/signals of the linear or rotational kinetic energy and the values of the linear or rotational kinetic power depend on the device in which the IMU 1 is arranged, e.g. on its moment of inertia I and/or its mass m. If this is a dedicated device which is always the same, this is just a constant and does not play a big role. However, when using a smartphone 5 or other wearable, the correct mass and/or moment of inertia must be retrieved for calculating the pre-feature signal(s), the features, the lin/rot kinetic energy and/or the lin/rot kinetic power. In this case, the processing means, preferably the monitoring app retrieves from the operating system of the smartphone 5 or the wearable, the type of device including the IMU 1. Based on the type of device, the mass and/or moment of inertia of the device can be retrieved. Thus, the kinetic energy and power is calibrated with the device characteristics measuring the motion of the heart and avoiding false results based on the use of different measuring devices. Subsequently, the term kinetic energy signal K(t) shall refer to the two-dimensional signal $K(t)=(K_{lin}(t), K_{rot}(t))$ containing the linear kinetic energy signal $K_{lin}(t)$ as a first dimension or component and the rotational kinetic energy signal $K_{rot}(t)$ as a second dimension or component or to one of the two components individually. Subsequently, the term kinetic power signal P(t) shall refer to the two-dimensional signal $P(t)=(P_{lin}(t), P_{rot}(t))$ containing the linear kinetic power signal $P_{lin}(t)$ as a first dimension or component and the rotational kinetic power signal $P_{rot}(t)$ as a second dimension or component or to one of the two components individually.

In step S34, at least one heartbeat is identified. Preferably, a plurality of heartbeats (for example all) are identified in the SCG. If a plurality of heartbeats have been identified, at least one is selected for the further analysis. A heartbeat is the time window related to one heartbeat of the heart. The heartbeat is the periodic cardiac cycle of the heart. The heartbeat is normally defined with respect to one characteristic point in the heartbeat, normally the R or the Q in the ECG signal. Since the present invention is preferably realized without any ECG signal, the at least one heartbeat is identified based on the SCG, preferably based on at least one pre-feature signal computed in step S33, preferably based on the kinetic energy signal and/or the kinetic power signal, or a combination of those. In a preferred embodiment, the (data) features are calculated based on the SCG (or a pre-feature signal) of one heartbeat, i.e. one cardiac cycle. This shall also include the case where a representative heartbeat is computed by averaging a plurality of heartbeats (preferably of the same breathing state). In a preferred embodiment, the heart health state is determined based on a plurality of heartbeats by selecting a plurality of heartbeats and detecting in steps S34 and S35 and S4 the health state for each heartbeat. A composite heart health state can then be computed by combining the heart health state from the individual heart beats. The state of the art normally did not calculate the feature vector based on a single heartbeat which reduced the robustness of the method. Some state-of-the-art documents propose to calculate the feature vector based on the data of one heartbeat, but those methods always require an ECG signal for detecting the heartbeat. This is one of the innovations of the present invention that the heartbeat can be identified from the SCG (without the use of an ECG signal). The heartbeat period is determined based on characteristics of the heartbeat in the SCG and/or a pre-feature signal obtained from the SCG (e.g. kinetic energy or power). While the heartbeat in the SCG and the pre-feature signals misses such a clear characteristic as in the ECG signal, a robust detection of the heartbeat period is difficult. A preferred embodiment to detect the heartbeat in the SCG is described subsequently.

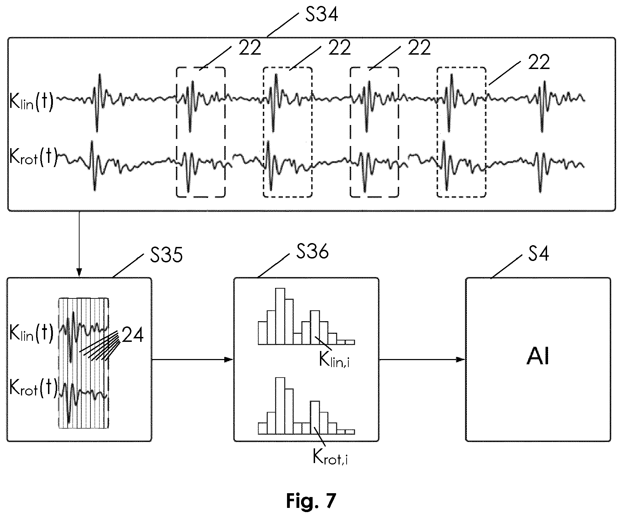

It is proposed to detect the heartbeat period in the SCG (including also in a pre-feature signal determined from the SCG) based on a matrix profile. The matrix profile m(t1) of a signal s(t) at the sample time t1 shows the distance of s(t1) to its nearest neighbor in the signal s(t) in the complete time window of the signal s(t). The distance is preferably the Euclidian distance metric, but obviously also other distance metrics can be use. The matrix profile m(t1) allows to find similar motifs in the signal s(t). Since the heartbeat of each person looks a bit different, this allows to find the repetitive motif of the heartbeat in the signal used for determining the matrix profile m(t). Preferably, the matrix profile m(t) is computed based on the SCG (including the meaning that the matrix profile m(t) is computed based on a (pre-feature) signal calculated from the SCG) and the heartbeat periods are determined computationally very quickly and with a high robustness. The matrix profile m(t) does not only provide the location of the motifs, i.e. the regular heartbeats, but also of data abnormalities called discords and the quality of each motif. The quality of each motif is the similarity measure to the most common motif in the signal s(t). Preferably, a multi-dimensional signal s(t) is used to detect the motifs/heartbeat periods. More details for an example of an algorithm to find the motifs in a multidimensional signal based on the matrix profile can be found in the article "Meaningful Multidimensional Motif Discovery" by Chin-Chia Michael Yeh, Nickolas Kavantzas, Eamonn Keogh published in ICDM 2017 which is incorporated by reference for exemplary details of the matrix profile. The motif length is preferably set to a value smaller than one second, preferably smaller than 800 miliseconds (ms), preferably smaller than 700 ms. The motif length is preferably set to a value larger than 200 ms, preferably larger than 400 ms, preferably larger than 500 ms. The motif length is preferably set to a value smaller than 1800 ms, preferably smaller than 1500 ms, preferably smaller than 1000 ms, preferably smaller than 800 ms. Fig. 7 shows an example of the motifs 22 detected with the matrix profile in the linear kinetic energy $K_{lin}(t)$ and in the rotation kinetic energy $K_{rot}(t)$ in step S34. In one embodiment, one motif 22 is detected. It is however also possible to detect two or more motifs 22 in the underlying signal as shown in Fig. 7.

The heartbeat period(s) is/are preferably identified based on a pre-feature signal, preferably based on the kinetic energy and/or power signal.

In step S35, sub-periods 24 of the heartbeat are identified in the identified heartbeat period. Preferably, sub-periods 24 are determined based on one characteristic point of the SCG (including also the meaning of a pre-feature signal determined based on the SCG) in the heartbeat period. Preferably, the characteristic point is the maximum rotational kinetic energy or the maximum rotational kinetic power in the heartbeat. Preferably, the sub-periods 24 are determined based on the time of the characteristic point and a fixed time relation between the sub-periods 24 of the heartbeat period. A fixed time relation can be an absolute fixed time relation or a relative fixed time relation. An absolute fixed time relation shall mean that the width of the sub-periods 24 and their time distances among have all a fixed absolute time relation (fixed absolute time value) and just the position of the sub-periods 24 with their fixed absolute time relation among them is positioned correctly based on the time of the characteristic point. A relative fixed time relation shall mean that the width of the sub-periods 24 and/or their time distances among have all a fixed relative time relation (fixed percentage). Thus, in addition to the correct positioning of the sub-periods 24, also the scaling must be adapted. The scaling could for example depend on the heartbeat frequency or the length of the selected heartbeat. The sub-periods 24 have preferably all the same (absolute or relative) width. The width of each sub-period 24 is preferably smaller than 200 ms, preferably than 150 ms, preferably than 140 ms, preferably than 130 ms, preferably than 120 ms, preferably than 110 ms, preferably than 105 ms and/or is preferably larger than 20 ms, preferably than 40 ms, preferably than 50 ms, preferably than 60 ms, preferably than 70 ms, preferably than 80 ms, preferably than 90 ms, preferably than 95 ms. Preferably, the width of each sub-period 24 is 100 ms. Preferably, the width of each sub-period 24 is preferably smaller than 30%, preferably than 25%, preferably than 20%, preferably than 15%, preferably than 10% of the heartbeat period/length and/or is preferably larger than 2%, preferably than 5%, preferably than 7%, preferably than 8%, preferably than 9% of the heartbeat period/length. The sub-periods 24 are preferably not-overlapping, i.e. there is no sample time which appears in two sub-periods 24. The sub-periods 24 are preferably arranged one next to the other so that there are no sample times between two sub-periods 24 which do not belong to any sub-period 24. Preferably, more than 5, preferably more than 6, preferably more than 7, preferably more than 8, preferably more than 9, preferably more than 10 sub-periods 24 are identified. Preferably, less than 20, preferably less than 15, preferably less than 14, preferably less than 13, preferably less than 12 sub-periods 24 are identified. Preferably, eleven sub-periods 24 are identified. Preferably, a first sub-period 24 is arranged such to cover the characteristic point. A first group of sub-periods 24 is arranged before the first sub-period 24 and a second (distinct) group of sub-periods 24 is arranged after the first sub-period 24. The first group comprises preferably less than the half of the sub-periods 24 and/or the second group comprises preferably more than the half of the sub-periods 24. Fig. 7 shows an example of step S35 for the arrangement of 11 sub-periods 24 of each 100 ms with four sub-periods 24 before the characteristic point, one on the characteristic point and 6 after the characteristic point. The cumulative period of all sub-periods 24 can be longer or shorter than the heartbeat period, i.e. can extent already in the previous and/or next heartbeat period or cannot cover the full heartbeat period.

In step S36, the (data) features (used as input in the AI engine 4) are computed. The (data) features comprise preferably at least one feature $K_i$ for each sub-period 24, wherein i indicates the index for the respective sub-period 24 going from the sub-period i=1 to the sub-period i=n, wherein n is the number of sub-periods in the heartbeat period. Preferably, the same physical (kinetic) variable is calculated for each sub-period. Preferably, the data features comprise one or more of the linear kinetic energy, the rotational kinetic energy, the linear kinetic power, the rotational kinetic power, the respiration information or state, the rotational cardiac work, the linear cardiac work of each respective sub-period. The linear/rotational kinetic energy of the sub-period can be one or more of the cumulative linear/rotational kinetic energy in the sub-period, the linear/rotational kinetic energy density in the sub-period, the maximum value of the linear/rotational kinetic energy in the sub-period, the time sample of the maximum value of the linear/rotational kinetic energy in the sub-period, the minimum value of the linear/rotational kinetic energy in the sub-period, the time sample of the minimum value of the linear/rotational kinetic energy in the sub-period or other values like the mean, median, variance, etc. of the linear/rotational kinetic energy in the sub-period. The cumulative linear/rotation energy of the sub-period means the integral of the linear/rotational energy over the sub-period. The linear/rotational kinetic energy density in the sub-period is the cumulative linear/rotational kinetic energy in the sub-period divided by the width of the sub-period (its time duration). The linear/rotational kinetic power of the sub-period can be one or more of the cumulative linear/rotational kinetic power in the sub-period, the maximum value of the linear/rotational kinetic power in the sub-period, the time sample of the maximum value of the linear/rotational kinetic power in the sub-period, the minimum value of the linear/rotational kinetic power in the sub-period, the time sample of the minimum value of the linear/rotational kinetic power in the sub-period or other values like the mean, median, variance, etc. of the linear/rotational kinetic power in the sub-period. The cumulative linear/rotation power of the sub-period means the integral of the linear/rotational power over the sub-period. The linear/rotational kinetic power density in the sub-period is the cumulative linear/rotational kinetic power in the sub-period divided by the width of the sub-period (its time duration). For brevity, linear/rotational shall mean in this paragraph that this applies for the linear variable like linear kinetic energy, linear kinetic power, etc. and/or for the rotational variable like rotational kinetic energy, rotational kinetic power, etc. These (data) features are computed based on the SCG signal in the heart period (or in the respective sub-periods), preferably based on the pre-feature signal(s) in the heart period (or in the respective sub-periods), preferably based on the linear/rotational kinetic energy signal in the heart period (or in the respective sub-periods) and/or based on the linear/rotational kinetic power signal in the heart period (or in the respective sub-periods). The step S33 for the calculation of the pre-feature signals can be realized also later, i.e. after steps S33 and S35, if not needed for these steps. The step S33 can also be omitted and be included in the step S36 of the calculation of the (data) features directly.

The respiration state of each sub-period is calculated based on the respiration signal or the respiration state in the sub-period determined in step S31. The respiration information/state could also be input only once for the complete heartbeat, instead of for each sub-period. In this case, it is preferred to select a heartbeat period which corresponds fully to (only) one breathing state.

The (data) features could comprise further the motif quality obtained in step S34. This provides a kind of deviation of the normal pattern of heartbeat of the user 10 and can be used as a further interesting input feature for the AI engine 4.

Fig. 3 describes a preferred embodiment for determining the data features fed into the AI engine 4. Obviously alternative embodiments for the (data) feature extraction are possible. For example, the sub-periods could extend over a plurality of heartbeats (two or more). For example, different feature variables are calculated for different sub-periods. The start and end point of the sub-periods can be detected based on characteristic points in the data. One, some or all features could be calculated for the complete heartbeat period instead of for the sub-periods. Many alternatives are possible.

Returning back to Fig. 2, the features determined in step S3 are fed in the AI engine 4 of the processing means 2. The AI engine 4 processes the features input and outputs a heart health state of the user 10. The heart health state preferably refers to one medical indication/problem of the heart. In a preferred embodiment, the heart health state refers to CHF. However, the discussed method could also be applied to detect other medical indications/problems of the heart like atrial fibrillation. Preferably, the heart health state is binary, i.e. the heart health state corresponds to a first heart health state indicating that the heart of the user 10 does not suffer the medical indication/problem, e.g. the CHF, or to a second heart health state indicating actually suffers or has a risk to suffer the medical indication/problem, e.g. CHF. Instead of a binary output of the AI engine 4, it is also possible to output a heart health state which can have more than two states. The second heart health state can be distinguished for example in different stages of the medical indication/problem or different risk ranges to have the medical indication/problem and/or also into different medical indications/problems. For CHF, the second heart health state could be sub-divided into different second substates indicating the stage of CHF like stage A, stage B, stage C, stage D. Thus, the output of the AI engine 4 would indicate not only the presence of CHF, but also its stage. It is further possible that the second heart health state could give out in addition a score indicating the probability of having this medical indication/problem.

The AI engine 4 is preferably a machine learning (ML) engine. The AI/ML engine 4 can be based on a random forest model, a deep learning model, neural network model, etc. In order to train the AI/ML engine 4, the same features used normally as input of the AI/ML engine 4 for users 10 for which the output, i.e. the heart health state is known. This known heart health state is input as well in the AI/ML engine 4 together with the features of the user 10 to train the AI/ML engine 4. This is done for many users 10 to obtain a well-trained AI/ML engine 4. In a deep learning model, some or all of the feature extraction steps can be incorporated in the deep learning model. This shall then still be considered as a feature extraction step S3 and a processing in an AI/ML engine 4 as in step S4 as described in this patent.

Preferably, the steps S3 and S4 are repeated for a plurality of heartbeats so that the heart health state is estimated for a plurality of heartbeats. The result of the heart health state of the plurality of heartbeats can be combined to obtain a combined heart health state. The combination can be for example to count the number of first heart health states and the number of second heart health states and if the percentage of second heart health states is above a threshold, the second heart health state is given out as combined heart health state. In case, a score is output with the second heart health state, this score can be used to improve the combination of the different heartbeat periods, e.g. by a weighted average. The step S3 might not be repeated completely for all heart periods. The steps S31 to S34 might be done once for the complete measurement window and only the steps S35 and S36 are repeated than for each heartbeat period which needs to be analyzed with the AI engine 4.

In a preferred embodiment, the feature extractor 3 and the AI engine 4 are arranged in the server 6 or the remote processor. Preferably, the local processor or the smartphone 5 sends (via a communication connection like internet) the measurement signal SCG received from the IMU 1 to the server 6 or remote processor for further processing (e.g. steps S 31 to S36 and S4). The output of the AI engine 4, i.e. the heart health state is preferably sent back to the local processor or smartphone 5 and is output to the user 10 via the output means of the smartphone 5. Obviously, it is also possible that the heart health state is sent elsewhere, e.g. to a healthcare professional (having requested the measurement), to an account of the user, or something else. However, it is also possible that a part of these steps or all steps are performed in the local processor or smartphone 5. If all steps are performed on the smartphone 5 or local processor, there are no problems for data privacy and the output from the AI engine 4 must not be sent back to the smartphone 5 or somewhere else.

It should be understood that the present invention is not limited to the described embodiments and that variations can be applied without going outside of the scope of the claims.

**Claims**

1. Method for detecting a health state of a heart of a user comprising the following steps:

   receiving, in a processing means, a measurement signal from an IMU placed on a body of the user,

determining, in the processing means, a plurality of features based on the measurement signal,

determine, in an AI engine of the processing means, based on the plurality of features the health state of the heart.

2. Method according to one of the previous claims, wherein the IMU is arranged in a smartphone, wherein the plurality of features is determined independently from an ECG measurement, wherein the measurement signal comprises a six-dimensional signal with three linear dimensions representing the linear acceleration of the heart measured with an accelerometer of the IMU and with three rotational dimensions representing the angular velocity of the heart measured with a gyroscope of the IMU, wherein a heartbeat period of the heart of the user is identified based on the measurement signal, wherein a plurality of sub-periods in the heartbeat period are determined, wherein the plurality of features comprise linear features and rotational features, wherein the linear features comprise the values of at least one linear parameter of the movement of the heart in each sub-period calculated based on the three linear dimensions of the measurement signal, wherein the rotational features comprise the values of at least one rotational parameter of the movement of the heart in each sub-period calculated based on the three rotational dimensions of the measurement signal.

3. Method according to the previous claim, wherein the at least one linear feature variable is one or more of: the linear power, the linear kinetic energy, and the linear work, and the at least one rotational feature variable is one or more of: the rotational power, the rotational kinetic energy, and the rotational work.

4. Method according to claim 2 or 3, wherein

a linear velocity signal is calculated based on the three linear dimensions and the at least one linear feature variable is calculated based on the linear velocity signal, and/or

a rotational acceleration signal is calculated based on the three rotational dimensions, and the at least one rotational parameter comprise at least one rotational feature calculated based on the rotational acceleration signal.

5. Method according to one of the previous claims, wherein a heartbeat period of the heart of the user is identified, wherein a plurality of sub-periods in the heartbeat period are determined, wherein the plurality of features comprise values of at least one feature variable in each determined sub-period, wherein a reference point in the heartbeat period is determined and the sub-periods are defined based on the reference point and based on fixed time widths of the sub-periods.

6. Method according to one of claims 2 to 5, wherein the heartbeat periods in the measurement signal are identified based on a matrix profile motif algorithm applied on a signal based on the measurement signal, wherein the matrix profile motif algorithm identifies one or more reference heartbeat periods as motif in the signal based on the matrix profile.

7. Method according to one of the previous claims, wherein a respiration information of the user is detected based on the measurement signal, wherein the plurality of features comprises a respiration information of the user.

8. Method according to one of the previous claims, wherein the plurality of features is determined independently from an ECG measurement.

9. Method according to one of the previous claims, wherein the AI engine determines, if the heart shows congestive heart failure.

10. Method according to one of the previous claims comprising the steps of placing the IMU on a chest of the user, and measuring the measurement signal with the IMU, when placed on the chest of the user.

11. Method according to the previous claim, wherein the IMU is included in a smartphone, wherein the processing means requests from an operating system running on the smartphone of what type the smartphone is, wherein the plurality of features depend on the type of smartphone received back.

12. Method according to the previous claim, wherein the user lays down, while a smartphone including the IMU is placed on the chest of the user.

13. System for detecting a health state of a heart of a user comprising:

an IMU, configured to measure a measurement signal when placed on a body of the user,
a processing means with an AI engine configured to perform the steps of the method according to one of claims 1 to 12 based on the measurement signal received from the IMU.

**14.** System according to the previous claim, wherein the system comprises a smartphone and a server, wherein the smartphone comprises the IMU and an application program running on a processor of the smartphone, wherein the application program is configured to connect with the server, wherein the processor of the smartphone and/or a processor of the server work as the processing means.

**15.** Computer program comprising instructions configured, when executed on a processing means, to perform the steps of the method according to one of claims 1 to 12.

2

10

3

SCG

Feature extractor

4

AI engine

**Fig. 1**

Measure SCG

S1

Receive SCG

S2

Determine features based on SCG

S3

Processes features in AI engine

S4

**Fig. 2**

Health
state

SCG

Determine
respiration
Information
S31

Preprocess SCG

S32

Determine pre-feature signals

S33

Determine heartbeat period

S34

Determine sub-periods of heartbeat

S35

Determine at least one feature for
each sub-period

S36

features

**Fig. 3**

**Fig. 4**

**Fig. 5**

**Fig. 6**

**Fig. 7**

Europäisches
Patentamt

European
Patent Office

Office européen
des brevets

## EUROPEAN SEARCH REPORT

Application Number

EP 22 16 5124

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/214030 A1 (MIGEOTTE PIERRE-FRANÇOIS [BE] ET AL) 2 August 2018 (2018-08-02) * figures 1-15 * * paragraph [0001] – paragraph [0095] * * paragraph [0115] – paragraph [0216] * * paragraph [0241] – paragraph [0280] * ----- | 1-15 | INV. G16H50/30 A61B5/00 G16H40/63 G16H40/67 A61B5/024 |
| X | LEE HYUNWOO ET AL: "An Enhanced Method to Estimate Heart Rate from Seismocardiography via Ensemble Averaging of Body Movements at Six Degrees of Freedom", SENSORS, vol. 18, no. 1, 15 January 2018 (2018-01-15), pages 1-12, XP055879701, DOI: 10.3390/s18010238 * the whole document, in particular the Abstract, the last paragraph on page 2 and pages 3-8 * ----- | 1-15 | |
| A | Tyler Marrs: "Introduction to Matrix Profiles. A Novel Data Structure for Mining Time... ¦ by Tyler Marrs ¦ Towards Data Science", , 11 October 2019 (2019-10-11), XP055960144, Retrieved from the Internet: URL:https://towardsdatascience.com/introduction-to-matrix-profiles-5568f3375d90 [retrieved on 2022-09-12] * the whole document * ----- | 1-14 | TECHNICAL FIELDS SEARCHED (IPC) G16H A61B |

The present search report has been drawn up for all claims

| Place of search | Date of completion of the search | Examiner |
|---|---|---|
| Munich | 13 September 2022 | Sasa Bastinos, Ana |

EPO FORM 1503 03.82 (P04C01)

## ANNEX TO THE EUROPEAN SEARCH REPORT
## ON EUROPEAN PATENT APPLICATION NO.

EP 22 16 5124

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2022

| Patent document cited in search report | | Publication date | Patent family member(s) | | Publication date |
|---|---|---|---|---|---|
| US 2018214030 | A1 | 02-08-2018 | CN | 108471987 A | 31-08-2018 |
| | | | EP | 3135194 A1 | 01-03-2017 |
| | | | EP | 3340878 A1 | 04-07-2018 |
| | | | HK | 1256818 A1 | 04-10-2019 |
| | | | US | 2018214030 A1 | 02-08-2018 |
| | | | WO | 2017036887 A1 | 09-03-2017 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 201736887 A
- US 20210057101 A
- EP 3267886 A
- EP 3897355 A
- EP 3895605 A
- EP 3731746 A

### Non-patent literature cited in the description

- **CHIN-CHIA MICHAEL YEH ; NICKOLAS KAVANTZAS ; EAMONN KEOGH.** Meaningful Multidimensional Motif Discovery. *ICDM,* 2017